(19) Europäisches Patentamt
European Patent Office
Office européen des brevets

(11) EP 2 819 669 B1

(12) **EUROPÄISCHE PATENTSCHRIFT**

(45) Veröffentlichungstag und Bekanntmachung des Hinweises auf die Patenterteilung:
**21.04.2021 Patentblatt 2021/16**

(21) Anmeldenummer: **13705805.3**

(22) Anmeldetag: **25.02.2013**

(51) Int Cl.:
***A61K 9/00*** *(2006.01)* ***A61K 47/12*** *(2006.01)*
***A61K 9/08*** *(2006.01)* ***A61K 31/439*** *(2006.01)*
***A61M 15/00*** *(2006.01)* ***A61P 11/00*** *(2006.01)*
***A61P 11/06*** *(2006.01)*

(86) Internationale Anmeldenummer:
**PCT/EP2013/053710**

(87) Internationale Veröffentlichungsnummer:
**WO 2013/127738 (06.09.2013 Gazette 2013/36)**

(54) **NEUE TREIBGASHALTIGE TIOTROPIUM-FORMULIERUNG**

NEW TIOTROPIUM FORMULA CONTAINING PROPELLANT

NOUVELLE FORMULATION DE TIOTROPIUM CONTENANT UN GAZ PROPULSEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.02.2012 EP 12157237**

(43) Veröffentlichungstag der Anmeldung:
**07.01.2015 Patentblatt 2015/02**

(73) Patentinhaber: **Boehringer Ingelheim International GmbH**
**55216 Ingelheim am Rhein (DE)**

(72) Erfinder:
- **BERNER, Bettina**
**55216 Ingelheim am Rhein (DE)**
- **HOELZ, Hubert**
**55216 Ingelheim am Rhein (DE)**
- **MANN, Mariola**
**55216 Ingelheim am Rhein (DE)**

(74) Vertreter: **Simon, Elke Anna Maria et al**
**Boehringer Ingelheim GmbH**
**Binger Strasse 173**
**55216 Ingelheim am Rhein (DE)**

(56) Entgegenhaltungen:
**EP-A1- 2 606 891** **WO-A1-02/38154**
**WO-A1-2005/042528** **US-A- 5 497 944**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft einen Inhalator enthaltend Druckgaszubereitungen für Dosieraerosole bei denen der Wirkstoff Tiotropiumbromid in HFA-Treibgasen (Hydro-Fluor-Alkane) als Treibmittel gelöst formuliert ist. Es handelt sich um ein Inhalationsaerosol.

## Stand der Technik

In der Behandlung von Krankheiten besteht Bedarf an Arzneimitteln, die aufgrund ihrer speziellen Dosierung geeignet sind, einerseits eine Krankheit zu behandeln (primäres therapeutisches Ziel) und andererseits potentielle Nebenwirkungen Minimal zu halten.

Es soll somit durch die Bereitstellung spezieller Dosierungen erreicht werden, dass eine medizinische Behandlung auf Basis einer patientenorientierten Entscheidung nach Möglichkeit auf der Grundlage von empirisch nachgewiesener Wirksamkeit unter Beachtung potentieller Nebenwirkungen getroffen werden kann. Folglich ist es Ziel einer derartigen Behandlung, dass die gewünschte Wirkung bei einem Minimum an Nebenwirkungen erreicht wird.

Im besonderen bedeutet eine solche Herangehensweise, dass für spezifische Patientengruppen, spezielle medikamentöse Therapien angeboten werden müssen (Patientenorientierte, indivualisierte Therapie).

Um derartige medizinische Ansätze im Rahmen von inhalativ zu verabreichenden Arzneimitteln verfolgen zu können, ist es - neben der medizinischen diagnostischen Einteilung und individuellen Zurordnung eines Patienten - erforderlich, dass genau abgegrenzte Dosierungen des Arzneimittels bereitgestellt werden, um somit sowohl ein "Optimum der Wirksamkeit" als auch an ein "Minimum der Nebenwirkung" bei der Behandlung eines Patienten zu erreichen. Für diese Zielsetzung ist die Dosis eines Inhalativum anhand von mehreren Dosisparametern (bevorzugt parallel durch mehrere Dosisparameter) zu definieren.

Lungenerkrankungen werden üblicherweise durch eine topische Applikation eines Wirkstoffes auf der Lungenoberfläche behandelt. Mittels solcher inhalativer Therapien werden dabei geringste Wirkstoffmengen durch Einatmen des Patienten weitestgehend gleichmäßig auf der Lungenoberfläche verteilt.

Somit weist die Behandlung von Erkrankungen der Lunge, welche durch eine topische medikamentöse Therapie mittels eines zu inhalierenden Aerosols erfolgt, spezielle Anforderungen an die Dosis-Genauigkeit einer topisch-inhalativen Wirkstoffgabe auf

Für die Behandlung von Lungenerkrankungen (z.B. COPD und Asthma), speziell für Chronisch Obstruktive Lungenerkrankungen wird der Wirkstoff Tiotropium **1**

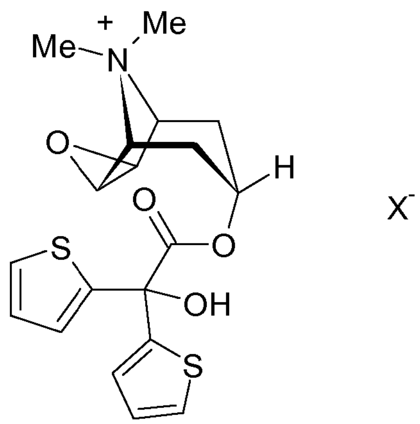

(**1**),

worin $X^-$ ein negativ geladenes Anion bedeutet, vorzugsweise ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, insbesondere Bromid (EP0418716), eingesetzt.

Die medikamentöse, topische Behandlung von Lungenerkrankungen mittels Tiotropium bedingt aufgrund der hohen Wirkstärke des Tiotropiums, dass eine täglich Gabe (in vivo) von wenigen μg ausreicht, um eine effektive Behandlung von COPD zu erzielen. Bei einer derartigen Behandlung werden durch Inhalation des Wirkstoffes ca. 0,5 μg bis 4 μg Substanz auf der Lungenoberfläche appliziert. Eine solche inhalative Applikation kann beispielsweise durch das Produkt SPIRIVA®/Handihaler® erfolgen. Bei einer geschätzten Lungenoberfläche von 70 $m^2$ und einer durchschnittlichen geometrischen Partikelgröße von 8$\mu m^3$ (weitere rechnerische Annahmen: Dichte von 3 $g/cm^3$, Lungendosis 3 μg) entspricht dies rechnerisch der Verteilung von 1 Mikropartikel pro 5-6 $cm^2$ Lungenoberfläche.

Da folglich bei der inhalativen Gabe eines Arzneimittels von der nominalen Dosis ausschließlich ein Bruchteil das Zielorgan "Lungenoberfläche" erreicht, beobachtet man daher nur eine sehr geringe Belegung der Lungenoberfläche (Partikel pro Lungenoberfläche) mit dem Wirkstoff, welche jedoch ausreicht, um einen therapeutischen Effekt zu erzielen.

Die Differenzmenge (im folgenden die Summe aus $X_{2a}$ und $X_{2b}$) zwischen nominaler Dosis *Y* und Lungendosis $X_1$ ist bei der inhalativen Verabreichung einesWirkstoffes nicht therapeutisch relevant in dem Sinne, dass die gewünschte therapeutische Wirkung erreicht wird, solange die Lungendosis $X_1$ im gewünschten Bereich liegt.

Jedoch ist die Differenz zwischen nominaler Dosis und Lungendosis relevant bezüglich der Fragestellungen, ob

(i) die Therapie wirtschaftlich oder unwirtschaftlich ist, und/oder ob sogar
(ii) mit einer erhöhten Nebenwirkungsrate zu rechnen ist.

Diese Differenzmenge zwischen nominaler Dosis *Y* und Lungendosis $X_1$ besteht wiederum aus einer Teilmenge, die bei der Ausbringung aus technischen Gründen beispielsweise im Ausbringunggerät zurückbleibt (Teilmenge $X_{2a}$) und folglich dem Patienten nicht zugeführt wird, sowie aus einer zweiten Teilmenge, die der Patient zwar mit dem Inhalieren des Produktes aufnimmt, jedoch nicht dem Zielorgang "Lungenoberfläche" zugeführt wird und folglich das Zielorgan "Lungenoberfläche" nicht erreicht (Teilmenge $X_{2b}$). Beispielsweise betrifft das den Wirkstoff-Anteil, der sich im Mund-Rachenraum abscheidet und anschließend durch den Patienten verschluckt wird.

Dabei gilt, dass im besonderen diese Teilmenge $X_{2b}$ für das Nebenwirkungsprofil verantwortlich ist, da der Wirkstoff Tiotropium, der aus therapeutischer Sicht ausschließlich topisch auf der "Lungenoberfläche" verfügbar sein soll / wirken soll, in Abhängigkeit der tatsächlichen Höhe der Teilmenge $X_{2b}$ dem Körper in anderer Art und Weise zur Verfügung gestellt wird. Dieser Wirkstoffanteil $X_{2b}$ der Dosis bedingt, dass unerwünschte Wirkungen des Wirkstoffes im Körper (Nebenwirkungen) beobachtbar sein können.

Zusammenfassend gilt folglich für die inhalative Gabe eines Wirkstoffes, dass die nominale Dosis *Y* durch die Applikation, d.h. dem Inhalationsvorgang durch den Patienten, funktional in eine Lungendosis $X_1$, eine Teilmenge Dosis, die dem Patienten nicht zur Verfügung steht (Verlust) $X_{2a}$ , welche beispielsweise im Inhalationsgerät verbleibt, und einer Teilmenge der Dosis, die der Patient aufnimmt, jedoch die nicht das Zielorgang "Lungenoberfäche" erreicht $X_{2b}$ , zu zerlegen ist, um die Dosis eine inhalativ applizierten Arzneimittels zu beschreiben. Ein optimales Verhältnis zwischen der verabreichten Teilmengen der Dosis des Wirkstoffes bewirkt hierbei eine Optimierung des Wirkung- und Nebenwirkungsprofils des Arzneimittels.

Es gilt für dis Dosis: $Y = X_1 + (X_{2a} + X_{2b})$

Die inhalative Applikation eines Wirkstoffes ist prinzipiell durch verschiedene pharmazeutische Techniken möglich. Techniken zur inhalativen Applikation eines Wirkstoffes umfassen dabei die Möglichkeiten, dass der Wirkstoff zum Beispiel aufbereitet als Pulverformulierung verabreicht wird (WO 02/30389) oder alternativ als Formulierung in Form einer Lösung, wobei die Verabreichung mit einem Zerstäuber (Handgerät Respimat®) erfolgt (WO 03/084519), dem Patienten zur Verfügung gestellt wird.

Als weitere dem Fachmann bekannte inhalative Applikationsform ist die Verabreichung einer inhalativen Gabe mittels eines Druckgas-betriebenen Dossieraerosols aufzuführen. Dabei wird eine Formulierung, in der der Wirkstoff in gelöster Form oder suspendiert vorliegt, durch die Beimischung eines verflüssigten Treibgases mittels eines Dosierventils portioniert, und in Folge der Abgabe aus dem Düsenkopf und der damit einhergehenden Entspannung in ein inhalierfähiges Aerosol überführt (WO 03/000241, EP2322243, WO2006/002840). Die WO 2005/042528 A1 offenbart Lungenaerosole enthaltend verschiedene Tiotropiumsalze, Ethanol/Wasser, Ascorbinsäure und das Treibgas HFA-134a.

Jede dieser speziellen inhalativen Applikationsformen bedingen spezielle Formulierungen für die jeweilige spezielle Darreichungstechnik (z.B. Pulverinhalation, Soft-Mist-Inhaler Respimat®, Treibgas-betriebenes Dosieraerosol). Es besteht aus pharmazeutischtechnologischer und medizinischer Sicht die Aufgabe, für die ausgewählte Darreichungstechnik, das Produkt / die Formulierung so zu optimieren, dass für eine aus medizinischer Sicht gewünschte Lungendosis $X_1$ ein optimiertes Verhältnis aus den Größen $X_1$ *zu* $Y$, $X_1$ zu $X_{2a}$ beziehungsweise $X_1$ zu $X_{2b}$ erreicht wird. Eine spezielle Aufgabe ist dabei, dass das Verhältnis $X_1$ zu $X_{2b}$ optimiert wird.

Insbesondere ist es Aufgabe der vorliegenden Erfindung, Inhalationsaerosolformulierungen für den Wirkstoff Tiotropium bromid bereitzustellen, in denen der Wirkstoff gelöst ist und damit mittels eines Treibgas-betriebenen Dosieraerols mit einer hohen Dosiergenauigkeit verabreicht werden kann, wobei im speziellen die Dosiergenauigkeit über die Laufzeit erhalten bleibt und folglich auch die Stabilität der Inhalationslösung in Abhängigkeit der Zeit gewährleistet ist.

## Definitionen

<u>Dosisparameter "nominale Dosis Y" :</u>

Unter der nominalen Dosis *Y* ist die metered dose zu verstehen. Die nominale Dosis entspricht der Menge an Wirkstoff, die durch die Applikation dem Patienten prinzipiell mit Hilfe des Inhalators pro einmaligen Auslösens desselben

zur Verfügung gestellt werden soll. Die nominale Dosis ist somit die Menge an Wirkstoff, die rechnerisch durch die Größen *Wirkstoff-Konzentration* und *Dosierkammer- Volumen* bestimmbar ist.

Dosisparameter "Lungendosis $X_1$":

Unter Lungendosis $X_1$ ist die Menge an Wirkstoff zu verstehen, die bei der Ausbringung des Wirkstoff als lungengängiges Aerosol zum Zwecke der Inhalation zur Verfügung steht. Die Lungendosis $X_1$ entspricht hierbei der *Fine Particle Dose* wie sie in den Arzneibüchern definiert ist. Gemäß der vorliegenden Erfindung wird die Lungendosis als Fine Particle Dose bestimmt (Pharm. Eur. 2.9.18, European Pharmacopeia, 6th Edition 2008, Apparatus D, Andersen Cascade Impacter) bzw. USP30-NE25 < 601 >).

Dosisparameter "Ausgebrachte Dosis *DD* ":

Die ausgebrachte Dosis (delivered dose *DD*) ist die Dosis, die vom Inhalator prinzipiell an den Patienten abgegeben wird. Die Bestimmung erfolgt gemäß dem Arzneibuch (Pharm Eur 0671, USP <601> Delivered Dose Apparatus A) auf Basis der Messung mit einer Ausbringeinheit. Das Dosis-Sammelrohr besitzt für die Messung einen für das verwendete Device (Mundstück) angepassten Adapter.

Dosisparameter "Teilmenge Dosis, die dem Patienten nicht zur Verfügung steht $X_{2a}$ " :

Erfindungsgemäß entspricht die Dosis $X_{2a}$ der nominalen Dosis *Y* abzüglich der ausgebrachten Dosis *DD.*
Es gilt: $X_{2a} = Y - DD$

Dosisparameter "Teilmenge der Dosis, die der Patient aufnimmt, jedoch die nicht das Zielorgang "Lungenoberfäche" erreicht $X_{2b}$" :

Die Teilmenge Dosis $X_{2b}$, die der Patient aufnimmt, jedoch die nicht das Zielorgang "Lungenoberfäche" erreicht, entspricht der ausgebrachten Dosis *DD* abzüglich der Lungendosis $X_1$.
Es gilt: $X_{2b} = DD - X_1$

Dosisparameter "mittlere aerodynamische Partikelgröße *MMAD* ":

Erfindungsgemäß ist unter der mittleren aerodynamischen Partikelgröße *MMAD* der aerodynamisch mittlere Massendurchmesser zu verstehen. Die Bestimmung dieser Größe erfolgt gemäß dem Arzneibuch (Pharm Eur 2.9.18 Apparatus D, USP USP <601> Aerodynamic size distribution Apparatus 1) auf Basis der Messung mit einem Anderson Kaskadenimpaktors. In Abhängigkeit der aerodynamischen Partikelgröße erfolgt die Abscheidung der Partikel in der Lunge in unterschiedlicher Lungentiefe (z.B. in den Bronchien, den kleinen Bronchien, oder den Lungenbläschen, etc.). Die gewählte aerodynamische Partikelgröße dient somit zur Steuerung, wo innerhalb der Lunge bevorzugt der Wirkstoff sedimentieren und somit zur Verfügung stehen soll.

"Lungenoberfäche ":

Die Lungenfläche stellt die innere Oberfläche der Luftwege in der Lunge dar. Sie umfasst in etwa 70 $m^2$.

Lösungsdosieraerosol:

Ein Dosieraerosol ist eine Darreichungsform für Wirkstoffe, die zur Inhalation durch den Patienten bestimmt sind. Bei einem Dosieraerosol wird der Wirkstoff in portionierten Dosen als Aerosol freigesetzt, d.h. als fein verteilte Flüssigkeitströpfchen in einer Gasphase. Die Abgabe einer Einzeldosis bezeichnet man als Sprühstoß.

Lösungsdosieraerosole enthalten den Wirkstoff in gelöster Form im Treibgas und einem Co-Solvent, wobei die Wirkstofflösung mit Hilfe von Treibgas vernebelt wird. Die Wirkstofflösung befindet sich in einem mit Treibgas gefüllten Medikamentenbehälter (Kartusche) mit Mundrohr, wobei auf Druck fein vernebelter Wirkstoff in abgemessener Dosierung freigesetzt wird.

Wirkung-/Nebenwirkungsprofil:

Eine Nebenwirkung ist eine neben der beabsichtigten Hauptwirkung auftretende Wirkung eines Arzneimittels, die jedoch im Sinne der vorliegenden Erfindung nicht das eigentliche Behandlungsziel (Hauptwirkung) des Arzneimittels

darstellt (eine unerwünschte Nebenwirkung wird im folgenden mit dem Begriff "Nebenwirkung" gleichgestellt). Eine solche Nebenwirkung kann gegebenfalls im Rahmen der Verabreichung eines Medikaments auftreten. Unter Wirkung eines Arzneimittels ist die therapeutisch beabsichtigte Hauptwirkung eines Arzneimittels zu verstehen. Man kann Nebenwirkungen unterteilen in (i) Arzneistofftypische und dosisabhängige Nebenwirkungen und (ii) dosisunabhängige Nebenwirkungen (etwa Überempfindlichkeitsreaktionen). Für inhalativ verabreichte Arzneimittel gilt hierbei, dass sowohl die Wirkung als auch dosisabhängige Nebenwirkungen (i) abhängig sind von den Eigenschaften / der Art der Formulierung sowie der speziellen Applikationsform (z.B. Dosieraerosol, Pulverinhalation, etc.). Demzufolge beobachtet man Wirkung sowie dosisabhängige Nebenwirkung in Abhängigkeit der spezifisch gewählten Kombination der nominalen Dosis *Y*, der Lungendosis $X_1$ und der ausgebrachte Dosis *DD,* sowie der Größen Dosis $X_{2a}$, Dosis $X_{2b}$ und der aerodynamischen Partikelgröße (vorallem des *MMADs*) sowie der gewählten Applikationsform.

Bestimmung Tiotropiumbromid-Gehalt in der Formulierung:

Der Gehalt von Tiotropium **1** in Form des Tiotropiumbromids kann mittels HPLC auf Basis einer isokratischen Methode bestimmt werden.

Parameter der Methode:

Säule: ACE 3 C18, 75 mm x 4,6 mm (Hersteller: ACE®)
Detektion: UV, 235nm
Fließmittel: 70% $KH_2PO_4$ Puffer, pH 3,0 / 30% Methanol
Probenvolumen: 10μl
Fluss: 1,5 ml/min

pH-Wert - Bestimmung:

Unter dem pH-Wert der treibgashaltigen Lösungsformulierungen ist der korrespondierende pH-Wert zu verstehen, der durch Umrechnung gemäß der Literaturstelle "Interpretation of pH Measurments in Alcohol-Water Solvents", J.Phys.Chem. Volume 67, Sept. 1963, Seite 1833-1838 ausgehend vom gemessenem Wert erhältlich ist. Der Messwert, der in die Umrechnung einfließt, kann dabei direkt mittels pH-Elektrode aus der ethanolischen Lösung, bzw. der Lösung des Wirkstoffes in dem/den Co-Solventien erhalten werden.

Methode zur Messung des pH-Wertes:

*pH-Elektrode*: Für pH-Messungen werden Glaselektroden (KCl-Elektrode) mit Brückenelektrolyt eingesetzt.Derartige pH-Elektroden sind ab Werk standardmäßig mit 3 molarer KCl-Lösung als Brückenelektrolyt befüllt und können direkt eingesetzt werden.
*Durchführung*: Die Messung wird bei Raumtemperatur (23°C +/- 2°C) durchgeführt.
Sofern vom Hersteller der pH-Elektrode nicht anders angegeben, wird zuerst der "Nullpunkt" [mV] der Elektrode mit Pufferlösung pH 7 ermittelt.
Hierzu wird bei nicht automatischen Geräten am pH-Meter der pH-Wert der Pufferlösung in Abhängigkeit der Temperatur eingestellt und der zugehörige Spannungswert [mV] und die Temperatur [°C] abgelesen. Zur Bestimmung der "Steilheit" wird ein zweiter Puffer, dessen Wert um mindestens 2 pH-Einheiten von pH 7 abweicht und den pH-Wert der zu messenden Probe umschließt, verwendet. Die Messung erfolgt wie in der Bedienungsanleitung des Herstellers des/der pH-Meters/pH-Elektrode angegeben. Vor dem Wechsel in eine neue Probe, Elektrode mit deionisiertem Wasser abspülen und Resttropfen vorsichtig abtupfen.
Das Ablesen des Messwertes (pH-Wert und parallele Bestimmung der Temperatur) erfolgt, sobald der Wert (mindestens 20 Sekunden).konstant (±0,05 pH-Einheiten) bleibt.

Normalität ("z.B. 0,01 normale Säure"):

Unter Angabe der Normalität (Einheitszeichen: N) wird die Äquivalentkonzentration $c_{eq}$ verstanden, die eine typische Konzentrationsangabe in der Chemie darstellt. Sie ist eine spezielle Stoffmengenkonzentration, der ein Bezugsfaktor 1/*z* zugrunde liegt. Hierbei ist z die stöchiometrische Wertigkeit, die auch Äquivalentzahl genannt wird. Im Falle z = 2 ist also die Äquivalentkonzentration doppelt so groß wie die Stoffmengenkonzentration, weil sozusagen jedes ganze Teilchen z-mal gezählt wird. Den Ausdruck 1/*z* nennt man auch Äquivalentteilchen oder Äquivalente. $c_{eq}$ ist ein

Maß dafür, wie viele Äquivalentteilchen eines Stoffes sich in einem bestimmten Volumen der Lösung befinden.

Die Normalität ist dann definiert durch die Gleichung:

$$N = n_{val} / V.$$

Hierbei ist *N* die Normalität, $n_{val}$ die Menge an Äquivalenten und *V* das Volumen.

Beispielsweise gilt für Schwefelsäure, dass an ein Sulfation ($SO_4^{2-}$) sich zwei Protonen anlagern können, was der Wertigkeit des Säure-Ions entspricht. Folglich sind in der Lösung doppelt so viele Äquivalentteilchen (hier Protonen) enthalten, wie Moleküle des Stoffes selbst.

$$H_2SO_4 \rightarrow SO_4^{2-} + H^+,$$

d. h., 1 mol/l ($H_2SO_4$) = 2 N ($H_2SO_4$) oder anders ausgedrückt, ist eine 1 normale $H_2SO_4$-Lösung ½ molar (1 N entspricht in diesem Beispiel ½ M).

**Beschreibung der Erfindung**

Die vorliegende Erfindung betrifft einen Inhalator enthaltend eine neuartige treibgashaltige Arzneimittelzusammensetzung auf Basis des Bromidsalzes von Tiotropium **1**, und dessen Eignung bei der Therapie von Atemwegserkrankungen.

Überraschenderweise kann ein unerwarteter vorteilhafter therapeutischer Effekt, insbesondere unter Beachtung einer verminderten Nebenwirkungsrate bei der Behandlung von entzündlichen oder obstruktiven Atemwegserkrankungen und/oder Asthma gezeigt werden, wenn Tiotropiumbromid als Lösungsdosieraerosol in einer spezifischen Dosierung zur Anwendung gelangt. Aufgrund dieser speziellen Dosierung sind die erfindungsgemäßen Tiotropium-haltigen Arzneimittelzusammensetzung im besonderen auch als Monotherapie einsetzbar, wobei das Wirkung-/Nebenwirkungsprofil durch die speziell anzuwendende Dosierung optimiert ist. Derartige Arzneimittelformulierungen zeichnen sich dabei erfindungsgemäß durch eine verbesserte pharmazeutische Stabilität (chemisch und physikalisch), vorallem auch durch eine optimale Stabilität unter gleichzeitiger Beibehaltung obiger Dosisanforderungen über die Zeit aus.

Im Rahmen der vorliegenden Erfindung werden unter Tiotropium **1** Salze verstanden. In den vorstehenden Salzen stellt das Kation Tiotropium den pharmakologisch wirksamen Bestandteil dar. Im Rahmen der vorliegenden Patentanmeldung ist eine explizite Bezugnahme auf vorstehende Kationen durch Verwendung der Bezeichnung **1'** erkennbar. Im Rahmen der vorliegenden Erfindung handelt es sich bei Tiotropium **1** um das Tiotropiumbromid.

Die Applikation von **1** erfolgt durch Erzeugung eines Inhalationsaersols durch Versprühen einer unter Druck stehenden Wirkstofflösung. Derartige Inhalationsaerosole enthalten ein Treibgas zur Erzeugung des Inhalationsaerosols. Die zur Herstellung von Inhaltionsaerosolen einsetzbaren Treibgase sind aus dem Stand der Technik bekannt. Die erfindungsgemäßen treibgashaltigen Inhalationsaerosole enthalten das Treibgas HFA 134a. In einer weiteren Ausführungsform können die erfindungsgemäßen treibgashaltigen Inhalationsaerosole neben dem Treibgas HFA 134a weitere Treibgase enthalten, die beispielsweise ausgewählt sind aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können als Zusatz zu dem Treibgas HFA 134a dabei allein oder in Mischungen neben dem Treibgas HFA 134a zur Anwendnung kommen. Erfindungsgemäß besonders bevorzugt sind treibgashaltigen Inhalationsaerosole, die als Treibgas ausschließlich HFA134a enthalten. Die erfindungsgemäßem treibgashaltigen Inhalationsaerosole können ferner weitere Bestandteile wie Kosolventien, Stabilisatoren, oberflächenaktive Mittel (surfactants), Antioxidantien, Schmiermittel sowie Konservierungsmittel enthalten. All diese weiteren Bestandteile sind im Stand der Technik bekannt.

Die erfindungsgemäßen Formulierungen zur Bereitstellung von treibgashaltigen Inhalationsaerosolen enthalten Mittel zur Einstellung des pH-Werts. Die erfindungsgemäßen treibgashaltigen Inhaltionsaerosole weisen einen pH-Wert von 6-8 auf, bevorzugt von 6,5 - 7,5. Dabei entspricht der pH-Wert der treibgashaltigen Inhaltionsaerosole erfindungsgemäß dem pH-Wert, der durch Umrechnung aus dem gemessenen Wert erhalten werden kann. Der für die Umrechnung verwendete gemessene Wert entspricht dem Wert, der mittels pH-Elektrode direkt in der Lösung des Wirkstoffes in dem/den Co-Solventien gemessen werden kann, nämlich in der Lösung aus Wirkstoff und Ethanol und Wasser.

Die treibgashaltigen Inhaltionsaerosole enthalten eine Säure, bevorzugt ausgewählt aus der Gruppe bestehend aus anorganischen oder organischen Säuren. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure. Bevorzugte anorganische Säuren sind Salzsäure, Schwefelsäure. Es können auch die Säuren verwendet werden, die bereits mit dem Wirkstoff ein Säureadditionssalz bilden. Erfindungsgemäß enthält das Aerosol Zitronensäure. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß wird Zitronensäure verwendet. Erfindungsgemäß enthalten die treibgashaltigen Inhaltionsaerosole Zitronensäure in einem Anteil von 0,005 - 0,1 %, besonders bevorzugt in einem Anteil von 0,05 - 0,1 %, besonders bevorzugt von 0,04 - 0,09 %, ganz besonders bevorzugt von 0,03 - 0,08 %, des weiteren besonders bevorzugt von 0,02 - 0,05 %.

In einer weiteren bevorzugten Ausführungsform enthalten die treibgashaltigen Inhaltionsaerosole eine anorganische Säure in einer Konzentration, der einer $H^+$-Ionen-Menge entspricht, die durch Zugabe eines 1-3% Anteils einer 0,01 normalen Säure erhältlich ist, bevorzugt eine anorganische Säure in einer Konzentration, der einer $H^+$-Ionen-Menge entspricht, die durch Zugabe eines 1,5-2,5% Anteils einer 0,01 normalen Säure erhältlich ist Anorganische Säuren sind in den Ansprüchen nicht gelistet.

In einer weiteren bevorzugten Ausführungsform enthalten die treibgashaltigen Inhaltionsaerosole Salzsäure und/oder Bromwasserstoffsäure, bevorzugt Salzsäure als anorganische Säure.

In einer weiteren bevorzugten Ausführungsform enthalten die treibgashaltigen Inhaltionsaerosole Schwefelsäure als anorganische Säure.

Die erfindungsgemäßen treibgashaltigen Inhaltionsaerosole enthalten 0,005-0,1 Gew.-% Zitronensäure. In einer weiteren bevorzugten Ausführungsform enthalten die treibgashaltigen Inhaltionsaerosole Ascorbinsäure als organische Säure.

Der Anteil des gelösten Arzneistoffs **1** an der fertigen Zubereitung (bezogen auf eine Dosierkammer von 50 µl) beträgt erfindungsgemäß 0,005 - 0,03 %. Bevorzugt enthalten die erfindungsgemäßen Formulierungen zur Bereitstellung von treibgashaltigen Inhalationsaerosolen 0,01 - 0,02 % des gelösten Arzneistoffs **1**. Desweiteren bevorzugt enthalten die erfindungsgemäßen Formulierungen zur Bereitstellung von treibgashaltigen Inhalationsaerosolen 0,02 % des gelösten Arzneistoffs **1**.

Die erfindungsgemäße Gesamtzusammensetzung der treibgashaltigen Inhalationsaerosole ergibt sich aus dem prozentualen Anteil des gelösten Wirkstoffes (Gew.-%) zuzüglich des prozentualen Anteils an Co-Solventien (Gew.-%), zuzüglich des prozentualen Anteils an Säure (Gew.-%), wobei der Restanteil zu 100 % durch das Treibgas abgedeckt ist. Sofern in den erfindungsgemäßen Inhalationsaerosolen weitere Bestandteile wie Stabilisatoren, oberflächenaktive Mittel (surfactants), Antioxidantien, Schmiermittel sowie Konservierungsmittel enthalten sind, betragen deren Menge einen Anteil von weniger als 2 % (Gew.-%), bevorzugt weniger als 1 %, wobei durch das Vorliegen dieser weiteren Komponenten der Anteil an Treibgas entsprechend reduziert ist. In einer speziellen Ausführungsform enthalten die erfindungsgemäßen Inhalationsaerosole ausschließlich Wirkstoff, Co-Solventien, Säure und Treibgas.

Die erfindungsgemäßen Arzneimittelformulierungen werden als Einzeldosis mittels eines Sprühstoßes verabreicht, indem eine Dosis aus der Dosierkammer durch Betätigen des Inhalators durch Austritt aus der Düse ausströmt. Die Dosierkammer weist ein Volumen zwischen 25 µl und 100 µl, bevorzugt 50 µl auf. Erfindungsgemäß beziehen sich die Angaben der Zusammensetzung der Arzneimittelformulierung (Anteils der Säure in Gew.-% und des Anteils des gelösten Wirkstoffes 1 in Gew.-%) zur Bestimmung der Dosis bevorzugt auf eine Dosiskammer mit einem Volumen von 50 µl, sofern keine weiteren Angaben gemacht sind. Durch entsprechende rechnerische Anpassung (z.B. Verdoppelung, Halbierung) sind dem Fachmann auch die entsprechenden Konzentrationen für die Anteile der Säure und des Wirkstoffes für eine Dosiskammer von z.B. 25 µl und 100 µl zugänglich und durch die Erfindung umfasst.

Als weitere erfindungsgemäße Ausführungsform erfolgt die Dosierung durch 2 direkt aufeinander folgende Sprühstöße, bevorzugt innerhalb von weniger als 10 Minuten, so dass die Gesamtdosis der Verabreichung von 2 Teildosen von jeweils 1 Sprühstoß entspricht. Bei dieser Ausführungsform der Erfindung bezieht sich die nominale Dosis sowie die daraus abzuleitenden weiteren erfindungsgemäßen Dosisangaben auf die gesamt verabreichte Menge der beiden Sprühstöße.

Diese Dosierung ist im besonderen Maße geeignet zur Behandlung einer mittelgradigen oder hochgradigen COPD.

Erfindungsgemäße Arzneimittelformulierungen, die als Einzeldosis verabreicht werden, wobei die die Dosierkammer ein Volumen von 25 µl aufweist, enthalten bevorzugt den gelösten Arzneistoff **1** zu einem Anteil von 0,01 - 0,1 %, besonders bevorzugt zu einem Anteil von 0,01 - 0,09 %, besonders bevorzugt zu einem Anteil von 0,016 - 0,09 %, besonders bevorzugt zu einem Anteil von 0,02 - 0,08 %, besonders bevorzugt zu einem Anteil von 0,02 - 0,04 %, besonders bevorzugt zu einem Anteil von 0,04 %. Erfindungsgemäße Arzneimittelformulierung, die als Einzeldosis verabreicht werden, wobei die die Dosierkammer ein Volumen von 50 µl aufweist, enthalten bevorzugt den gelösten Arzneistoff **1** zu einem Anteil von 0,005 - 0,03 %, besonders bevorzugt zu einem Anteil von 0,01 - 0,02 %,besonders bevorzugt zu einem Anteil von 0,012 %, besonders bevorzugt zu einem Anteil von 0,02 %. Erfindungsgemäße Arzneimittelformulierung, die als Einzeldosis verabreicht werden, wobei die die Dosierkammer ein Volumen von 100 µl aufweist, enthalten bevorzugt den gelösten Arzneistoff **1** zu einem Anteil von 0,0025 - 0,025 %, besonders bevorzugt zu einem Anteil von 0,0025 - 0,0225 %, besonders bevorzugt zu einem Anteil von 0,004 - 0,02 %, besonders bevorzugt zu einem

Anteil von 0,005 - 0,02 %, besonders bevorzugt zu einem Anteil von 0,005 - 0,01 %, besonders bevorzugt zu einem Anteil von 0,01 %.

In einer weiteren besonders bevorzugten Form enthalten erfindungsgemäße Arzneimittelformulierungen ausschließlich den Arzneistoff **1** als einzige Komponente als pharmakologisch wirksamen Bestandteil (Wirkstoff) zur Behandlung von entzündlichen oder obstruktiven Atemwegserkrankungen.

Bei den im Rahmen der vorliegenden Erfindung genannten prozentualen Angaben, handelt es sich stets um Gewichtsprozente. Werden Massenanteile für Tiotropium in Gewichtsprozenten zum Ausdruck gebracht, sind die entsprechenden Werte für das im Rahmen der Herstellung der vorliegenden Erfindung bevorzugt zum Einsatz gelangende kristalline Tiotropiumbromid-Monohydrat durch Multiplikation mit dem Umrechnungsfaktor 1,2495 bezogen auf die molekulare Masse des Tiotropiumbromids erhältlich.

Die Formulierungen zur Bereitstellung von treibgashaltigen Inhalationsaerosolen enthalten erfindungsgemäß Co-Solventien. Erfindungsgemäß enthalten die Formulierungen eine Mischung aus Wasser und Ethanol als Co-Solventien. Erfindungsgemäß enthalten die Formulierungen einen Wasseranteil von 0,5 - 2,5 % und 15 - 40 % Ethanol. Besonders bevorzugt enthalten die Formulierungen einen Wasseranteil von 0,5 - 2 % und 15 - 40 % Ethanol. Darüberhinaus bevorzugt enthalten die Formulierungen einen Wasseranteil von 1 - 2 % und 15 - 35 % Ethanol. Weiterhin bevorzugt enthalten die Formulierungen einen Wasseranteil von 1 - 2 % und 20 - 30 % Ethanol. Erfindungsgemäß werden den Treibgasen oder den Lösungen aus dem Arzneistoff **1** und den Co-Solventien bevorzugt diese Mengen Wasser zugesetzt, wenn das Treibgas, Treibgasgemisch oder die Formulierung kein sonstiges Wasser enthält (freies Wasser). Verfahrenstechnisch kann dabei das Wasser bereits dem Treibgas zugemischt werden, bevor die Arzneimittelformulierung hergestellt wird oder zunächst wird die Arzneimittelformulierung mit wasserfreiem Treibgas oder Treibgasgemisch hergestellt und anschließend wird die entsprechende Menge Wasser beigemischt.

Gegebenenfalls wird im Rahmen der vorliegenden Erfindung statt des Begriffs Lösungsformulierung auch der Begriff Arzneimittelformulierung verwendet. Im Rahmen der vorliegenden Erfindung sind diese Begriffe als gleichbedeutend anzusehen.

Die erfindungsgemäßem treibgashaltigen Inhalationsaerosole bzw. Lösungsformulierungen können ferner weitere Bestandteile wie oberflächenaktive Mittel (Tenside, Surfactants), Adjuvantien, Antioxidantien oder Geschmacksmittel enthalten.

Die in den erfindungsgemäßen Lösungen gegebenenfalls enthaltenen oberflächenaktiven Mittel (Tenside, Surfactants) sind vorzugsweise ausgewählt aus der Gruppe bestehend aus Polysorbat 20, Polysorbat 80, Myvacet 9-45, Myvacet 9-08, Isopropylmyristat, Ölsäure, Propylenglycol, Polyethylenglycol, Brij, Ethyloleat, Glyceryltrioleat Glycerylmonolaurat, Glycerylmonooleat, Glycerylmonosterat, Glycerylmonoricinoleate, Cetylalcohol, Oleyloleat, Sterylalkohol, Cetylpyridinumchlorid, Blockpolymere, natürliches Öl und Isopropanol.

Sofern in den erfindungsgemäßen Lösungen oberflächenaktive Mittel enthalten sind, werden diese vorzugsweise in einem Anteil von 0,0005 - 1 %, besonders bevorzugt 0,005 - 0,5 % eingesetzt. In einer weiteren bevorzugten Ausführungsform enthalten die erfindungsgemäßen Lösungen keine oberflächenaktiven Mittel (Tenside, Surfactants).

Die in den erfindungsgemäßen Lösungen gegebenenfalls enthaltenen Antioxidantien sind vorzugsweise ausgewählt aus der Gruppe bestehend aus Ascorbinsäure, Zitronensäure, Natriumedetat, Editinsäure, Tocopherolen, Butylhydroxytoluol, Butylhydroxyanisol und Ascorbylpalmitat, wobei Tocopherole, Butylhydroxytoluol, Butylhydroxyanisol oder Ascorbylpalmitat bevorzugt zum Einsatz gelangen.

Die in den erfindungsgemäßen Lösungen gegebenenfalls enthaltenen Geschmacksmittel sind vorzugsweise ausgewählt aus der Gruppe bestehend aus Pfefferminz, Sacharin, Dentomint®, Aspartam und etherischen Ölen (beispielsweise Zimt, Anis, Menthol, Campher), wobei beispielsweise Pfefferminz oder Dentomint® besonders bevorzugt sind.

Zur Herstellung der erfindungsgemäßen Lösungen kann nach im Stand der Technik bekannten Verfahren vorgegangen werden. Hierzu werden die Bestandteile der Formulierung mit dem oder den Treibgasen (ggf. bei niedrigen Temperaturen) gemischt und in geeignete Behälter abgefüllt.

Überraschenderweise wurde gefunden, dass die Aufgabe der Erfindung gelöst wird durch die Bereitstellung eines Inhalators enthaltend ein Medikament zur Behandlung von entzündlichen oder obstruktiven Atemwegserkrankungen in Form einer Arzneimittelformulierung, wobei die Arzneimittelformulierung als Wirkstoff ausschließlich **1** in gelöster Form enthält, dadurch gekennzeichnet, dass die Arzneimittelformulierung das Treibgas HFA 134a sowie als weitere Bestandteile 0,005-0,03 % des Arzneistoffs **1**, eine Mischung aus den beiden Co-Solventien Ethanol und Wasser, wobei der Ethanol-Gehalt zwischen 15-40 % und der Wassergehalt zwischen 0,5-2,5 % beträgt, und Zitronensäure in einer Konzentration zwischen 0,005-0,1 Gew.-%.

In einer speziellen Ausführungsform wird die Aufgabe der Erfindung gelöst durch einen Inhalator enthaltend eine Arzneimittelformulierung zur Behandlung von entzündlichen oder obstruktiven Atemwegserkrankungen, wobei die Arzneimittelformulierung als Wirkstoff ausschließlich **1** in gelöster Form enthält, und dadurch gekennzeichnet ist, dass die Arzneimittelformulierung das Treibgas HFA 134a, sowie als weitere Bestandteile 0,005-0,03 % des Arzneistoffs **1**, eine Mischung aus den beiden Co-Solventien Ethanol und Wasser, wobei der Ethanol-Gehalt zwischen 15-40 % und

der Wassergehalt zwischen 0,5-2,5 % beträgt, und Zitronensäure in einer Konzentration zwischen 0,005-0,1 %, bevorzugt zwischen 0,008-0,09%, des weiteren bevorzugt zwischen 0,01-0,08, des weiteren bevorzugt zwischen 0,02-0,06, sowie des weiteren bevorzugt zwischen 0,02-0,05% enthält.

In einer speziellen Ausführungsform wird die Aufgabe der Erfindung gelöst durch eine Arzneimittelformulierung zur Behandlung von entzündlichen oder obstruktiven Atemwegserkrankungen, wobei die Arzneimittelformulierung als Wirkstoff ausschließlich **1** in gelöster Form enthält, und dadurch gekennzeichnet ist, dass die Arzneimittelformulierung das Treibgas HFA 134a, sowie als weitere Bestandteile 0,005-0,03 % des Arzneistoffs **1**, eine Mischung aus den beiden Co-Solventien Ethanol und Wasser, wobei der Ethanol-Gehalt zwischen 15-40 % und der Wassergehalt zwischen 0,5-2,5 % beträgt, und eine anorganische Säure in einer Konzentration, der einer $H^+$-Ionen-Menge entspricht, die durch Zugabe eines 1-3% Anteils einer 0,01 normalen Säure erhältlich ist, bevorzugt eine anorganische Säure in einer Konzentration, der einer $H^+$-Ionen-Menge entspricht, die durch Zugabe eines 1,5-2,5% Anteils einer 0,01 normalen Säure erhältlich ist, enthält Ausführungsformen, die keine Zitronensäure in der beanspruchten Menge enthalten, sind nicht Bestandteil der Erfindung. Entsprechende Mengen an anorganischen Säure, die der erfinderischen $H^+$-Ionen-Menge entspricht, lassen sich durch Zugabe von 1-3% einer 0,01 normalen Säure oder 0,1-0,3% einer 0,1 normalen Säure oder durch Zugabe einer entsprechenden Menge, die durch Umrechnung in vergleichbarer Art und Weise bestimmbar ist, erzielen. Entsprechende Mengen an anorganischen Säure, die der erfinderischen bevorzugten $H^+$-Ionen-Menge entspricht, lassen sich durch Zugabe von 1,5-2,5% einer 0,01 normalen Säure oder 0,15-0,25% einer 0,1 normalen Säure oder durch Zugabe einer entsprechenden Menge, die durch Umrechnung in vergleichbarer Art und Weise bestimmbar ist, erzielen.

In einer speziellen Ausführungsform betrifft die Erfindung einen Inhalator enthaltend eine Arzneimittelformulierung mit den zuvor oder danach genannten Eigenschaften, die ferner dadurch charakterisiert ist, dass die Arzneimittelformulierung als Säure Zitronensäure in einer Konzentration zwischen 0,005-0,1 %, bevorzugt zwischen 0,008-0,09%, des weiteren bevorzugt zwischen 0,01-0,08, des weiteren bevorzugt zwischen 0,02-0,06, sowie des weiteren bevorzugt zwischen 0,02-0,05% enthält.

In einer speziellen Ausführungsform, die nicht beansprucht wird, betrifft die Erfindung eine Arzneimittelformulierung mit den zuvor oder danach genannten Eigenschaften, die ferner dadurch charakterisiert ist, dass die Arzneimittelformulierung als Säure eine organische Säure, bevorzugt Ascorbinsäure in einer Konzentration zwischen 0,005-0,1%, bevorzugt zwischen 0,008-0,09%, des weiteren bevorzugt zwischen 0,01-0,08, des weiteren bevorzugt zwischen 0,02-0,06, sowie des weiteren bevorzugt zwischen 0,02-0,05% enthält.

In einer weiteren speziellen Ausführungsform, die nicht beansprucht wird, betrifft die Erfindung eine Arzneimittelformulierung mit den zuvor oder danach genannten Eigenschaften, die ferner dadurch charakterisiert ist, dass die Arzneimittelformulierung als Säure eine anorganische Säure in einer Konzentration, der einer $H^+$-Ionen-Menge entspricht, die durch Zugabe eines 1-3% Anteils einer 0,01 normalen Säure erhältlich ist, bevorzugt eine anorganische Säure in einer Konzentration, der einer $H^+$-Ionen-Menge entspricht, die durch Zugabe eines 1,5-2,5% Anteils einer 0,01 normalen Säure erhältlich ist, enthält.

In einer weiteren speziellen Ausführungsform, die nicht Teil der Ansprüche ist, betrifft die Erfindung eine Arzneimittelformulierung mit den zuvor oder danach genannten Eigenschaften, die ferner dadurch charakterisiert ist, dass die Arzneimittelformulierung als Säure wässrige Salzsäure in einer Konzentration, der einer $H^+$-Ionen-Menge entspricht, die durch Zugabe eines 1-3% Anteils einer 0,01 normalen Säure erhältlich ist, bevorzugt eine anorganische Säure in einer Konzentration, der einer $H^+$-Ionen-Menge entspricht, die durch Zugabe eines 1,5-2,5% Anteils einer 0,01 normalen Säure erhältlich ist, bevorzugt dass die Arzneimittelformulierung einen 1-3% Anteil einer 0,01 molaren Salzsäure, weiter bevorzugt, dass die Arzneimittelformulierung einen 1,5-2,5% Anteil einer 0,01 molaren Salzsäure enthält.

In einer weiteren speziellen Ausführungsform, die nicht Teil der Ansprüche ist, betrifft die Erfindung eine Arzneimittelformulierung mit den zuvor oder danach genannten Eigenschaften, die ferner dadurch charakterisiert ist, dass die Arzneimittelformulierung als Säure wässrige Schwefelsäure in einer Konzentration, der einer $H^+$-Ionen-Menge entspricht, die durch Zugabe eines 1-3% Anteils einer 0,01 normalen Säure erhältlich ist, bevorzugt eine anorganische Säure in einer Konzentration, der einer $H^+$-Ionen-Menge entspricht, die durch Zugabe eines 1,5-2,5% Anteils einer 0,01 normalen Säure erhältlich ist, enthält.

Die vorliegende Erfindung betrifft einen Inhalator enthaltend eine Arzneimittelformulierung mit den zuvor oder danach genannten Eigenschaften, die ferner dadurch charakterisiert ist, dass die nominale Dosis der Einmalgabe zwischen 5,5µg und 6,5µg, die ausgebrachte Dosis zwischen 4,5-5,6 µg, und die Lungendosis zwischen 2-3 µg und die mittlere aerodynamische Partikelgröße zwischen 2,5-3,5 µm beträgt.

In einer weiteren speziellen Ausführungsform betrifft die Erfindung eine Arzneimittelformulierung mit den zuvor oder danach genannten Eigenschaften, die ferner dadurch charakterisiert ist, dass die nominale Dosis der Einmalgabe zwischen 5,5µg und 6,5µg, und die ausgebrachte Dosis bevorzugt zwischen 4,7-5,4 µg, besonders bevorzugt zwischen 4,8-5,3 µg, des weiteren besonders bevorzugt zwischen 4,9-5,2 µg, sowie ganz besonders bevorzugt zwischen 5-5,1 µg beträgt.

In einer weiteren speziellen Ausführungsform betrifft die Erfindung eine Arzneimittelformulierung mit den zuvor oder danach genannten Eigenschaften, die ferner dadurch charakterisiert ist, dass die nominale Dosis der Einmalgabe

zwischen 5,5μg und 6,5μg, und die Lungendosis bevorzugt zwischen 2,0-3,0 μg, besonders bevorzugt zwischen 2,1-3,0 μg, ganz besonders bevorzugt zwischen 2,3-2,9 μg, und des weiteren ganz besonders bevorzugt zwischen 2,4-2,7 μg, beträgt.

In einer weiteren speziellen Ausführungsform betrifft die Erfindung eine Arzneimittelformulierung mit den zuvor oder danach genannten Eigenschaften, die ferner dadurch charakterisiert ist, dass die nominale Dosis der Einmalgabe zwischen 5,5μg und 6,5μg, und die mittlere aerodynamische Partikelgröße bevorzugt zwischen 2,6-3,4 μm, des weiteren bevorzugt zwischen 2,7-3,3 μm beträgt

Die Erfindung ist ferner charakterisiert durch einen Inhalator enthaltend eine Arzneimittelformulierung mit den zuvor oder danach genannten Eigenschaften, die ferner dadurch charakterisiert ist, dass die Dosis durch Abgabe einer Einzeldosis mittels Sprühstoß erfolgt, indem eine Dosis aus der Dosierkammer durch Betätigen des Inhalators durch Austritt aus der Düse ausströmt und das dadurch gebildete Aerosol gekennzeichnet ist, dass die nominale Dosis der Einmalgabe zwischen 5,5μg und 6,5μg, die ausgebrachte Dosis zwischen 4,5-5,6 μg, und die Lungendosis zwischen 2-3 μg und die mittlere aerodynamische Partikelgröße zwischen 2,5-3,5 μm beträgt.

Der Bohrungsdurchmesser des verwendeten Sprühkopfes besitzt einen Durchmesser zwischen 0,2-0,3 mm, bevorzugt zwischen 0,20-0,27 mm, ganz bevorzugt zwischen 0,20 und 0,25 mm, und das gebildete Aerosol ist dadurch gekennzeichnet, dass die nominale Dosis der Einmalgabe zwischen 5,5μg und 6,5μg, die ausgebrachte Dosis zwischen 4,5-5,6 μg, und die Lungendosis zwischen 2-3 μg und die mittlere aerodynamische Partikelgröße zwischen 2,5-3,5 μm beträgt.

Die Dosierkammer besitzt ein Volumen zwischen 25 μl und 100 μl, bevorzugt 50 μl.

In einer weiteren speziellen Ausführungsform betrifft die Erfindung eine Arzneimittelformulierung mit den zuvor oder danach genannten Eigenschaften, die ferner dadurch charakterisiert ist, dass die Dichtungsringe im Ventilkopf aus Ethylen-Propylen-Dien-Monomer (EPDM) hergestellt sind, und dass das gebildete Aerosol gekennzeichnet ist, dass die nominale Dosis der Einmalgabe zwischen 5,5μg und 6,5μg, die ausgebrachte Dosis zwischen 4,5-5,6 μg, und die Lungendosis zwischen 2-3 μg und die mittlere aerodynamische Partikelgröße zwischen 2,5-3,5 μm beträgt.

In einer weiteren speziellen Ausführungsform betrifft die Erfindung den erfindungsgemässen Inhalator enthaltend die Tiotropiumbromid-haltige Arzneimittelformulierung zur Verwendung bei der Behandlung von entzündlichen oder obstruktiven Atemwegserkrankungen, wobei die Arzneimittelformulierung durch die zuvor oder danach genannten Eigenschaften gekennzeichnet ist.

In einer weiteren speziellen Ausführungsform betrifft die Erfindung den Wirkstoff Tiotropium 1, welcher in einer der oben oder unten genannten Arzneimittelformulierung vorliegt, zur Verwendung als Medikament zur Behandlung von entzündlichen oder obstruktiven Atemwegserkrankungen, wobei das Medikament

- 1x täglich,
- mit einer nominalen Dosis zwischen 5,5μg und 6,5μg,
- mit einer ausgebrachten Dosis zwischen 4,5-5,6 μg, und
- mit einer Lungendosis zwischen 2-3 μg

verabreicht wird, wobei die mittlere aerodynamische Partikelgröße zwischen 2,5-3,5 μm beträgt Eine solche Verwendung ist nicht Teil der Ansprüche.

In einer weiteren speziellen Ausführungsform betrifft die Erfindung den Wirkstoff Tiotropium **1**, welcher in einer der oben oder unten genannten Arzneimittelformulierung vorliegt zur Verwendung als Medikament zur Behandlung von entzündlichen oder obstruktiven Atemwegserkrankungen, wobei das Medikament

- 1x täglich,
- mit einer nominalen Dosis zwischen 5,5μg und 6,5μg,
- mit einer ausgebrachten Dosis zwischen 4,5-5,6 μg und
- mit einer Lungendosis zwischen 2,0-3,0 μg, bevorzugt zwischen 2,1-3,0 μg, besonders bevorzugt zwischen 2,3-2,9 μg, und ganz besonders bevorzugt zwischen 2,4-2,7 μg

verabreicht wird, wobei die mittlere aerodynamische Partikelgröße zwischen 2,5-3,5 μm beträgt Eine solche Verwendung ist nicht Teil der Ansprüche.

In einer weiteren speziellen Ausführungsform betrifft die Erfindung den Wirkstoff Tiotropium **1**, welcher in einer der oben oder unten genannten Arzneimittelformulierung vorliegt zur Verwendung als Medikament zur Behandlung von entzündlichen oder obstruktiven Atemwegserkrankungen, wobei das Medikament

- 1x täglich,
- mit einer nominalen Dosis zwischen 5,5μg und 6,5μg,
- mit einer ausgebrachten Dosis zwischen 4,7-5,4 μg, bevorzugt zwischen 4,8-5,3 μg, besonders bevorzugt zwischen

4,9-5,2 μg, sowie ganz besonders bevorzugt zwischen 5-5,1 μg und

- mit einer Lungendosis zwischen 2-3 μg

verabreicht wird, wobei die mittlere aerodynamische Partikelgröße zwischen 2,5-3,5 μm beträgt Eine solche Verwendung ist nicht Teil der Ansprüche.

Die vorstehend genannten treibgashaltigen Lösungen werden erfindungsgemäss mittels im Stand der Technik bekannten Inhalatoren (pMDIs = pressurized metered dose inhalers) appliziert. Dementsprechend betrifft die Erfindung Arzneimittel in Form von wie vorstehend beschriebenen Lösungen in Verbindung mit einem oder mehreren zur Verabreichung dieser Lösungen geeigneten Inhalatoren. Ferner betrifft die vorliegende Erfindung Inhalatoren, dadurch gekennzeichnet, dass sie vorstehend beschriebene erfindungsgemäße treibgashaltige Lösungen enthalten. Die vorliegende Erfindung betrifft ferner Behälter (z.B. Kartuschen), die ausgestattet sind mit einem geeigneten und vor der Verwendung bzgl. des Wassergehaltes konditionierten Ventils. Die Behälter können in einem geeigneten Inhalator zur Anwendung gelangen und eine der vorstehend genannten erfindungsgemäßen treibgashaltigen Lösungen enthalten. Geeignete Behälter (z.B. Kartuschen) und Verfahren zur Abfüllung dieser Kartuschen mit den erfindungsgemäßen treibgashaltigen Lösungen sind aus dem Stand der Technik bekannt.

In der Kartusche (siehe Abbildung 1, Bezugszeichen "a") befindet sich das Medikament als Lösung. In der Formulierung sind neben dem Arzneistoff **1** auch andere chemische Substanzen vorhanden, welche für die Stabilität und Produktperformance wichtig sind. Über dem Flüssigkeitsspiegel ist weiteres gasförmiges Treibgas vorhanden. Das Treibgas in der Dosierkammer bewirkt das explosive Ausströmen der Arzneimittelformulierung aus derselben bei Betätigen des Dosieraerosols. (Im Sinne der vorliegenden Erfindung ist Kartusche gleichbedeutend zu Kanister).

In der Düse (siehe Abbildung 1, Bezugszeichen "b") einstehen die kleinen Flüssigkeitströpfchen, welche das Medikament enthalten. Die Grösse dieser Tröpfchen ist wichtig, um eine gute Deposition in der Lunge zu erreichen. Die Düse ist dabei gekennzeichnet durch Bohrungsdurchmesser (engster Durchmesser) des verwendeten Sprühkopfes "b".

Die Menge des ausgestossenen Medikamentes wird durch die Dosierkammer (siehe Abbildung 2, Bezugszeichen "c") dosiert.

Die Tröpfchengröße eines Dosieraerosols kann durch das Düsendesign (Bohrungsdurchmesser des Sprühkopfes, Größe der Dosierkammer) und durch die Zusammensetzung der Lösungsformulierung beeinflusst werden. Im speziellen besteht die Möglichkeit, die Tröpfchengröße durch die Wahl des Treibgases zu beeinflussen. Je kleiner der Düsendurchmesser des Sprühkopfes, desto kleiner werden die erzeugten Tröpfchen - hier sind dem Design allerdings Grenzen gesetzt, da zu kleine Düsen leicht verstopfen.

Die untere Grenze für die Bohrung liegt bei 0,16 mm, da eine kleinere Düse leicht verstopfen kann Erfindungsgemäss weist der Bohrungsdurchmesser des verwendeten Sprühkopfes einen Durchmesser zwischen 0,2-0,3 mm auf.

Die Verwendung eines Sprühkopfes, der einen Bohrungsdurchmesser zwischen 0,20-0,27 mm aufweist, ist im Rahmen der vorliegenden Erfindung besonders bevorzugt. Ebenso erfindungsgemäss ist die Verwendung einer Dosierkammer mit einem Volumen zwischen 25 μl und 100 μl, besonders bevorzugt 50 μl. Für die erfindungsgemäßen Dosieraerosole können kommerziell erhältliche Mundrohre verwendet werden (z.B. Hersteller RPC Formatec GmbH, Deutschland).

Ein weiterer Gegenstand der vorliegenden Erfindung, der nicht in den Ansprüchen verhaftet ist, betrifft ein Verfahren zur Dosierung eines Medikaments einer erfindungsgemäßen Formulierung zur Bereitstellung von treibgashaltigen Inhalationsaerosolen umfassend die Schritte

- Deckel abnehmen
- Kräftig schütteln, Kanister muss nach oben schauen
- ausatmen
- Dosieraerosol zwischen die Lippen nehmen
- langsam einatmen und sofort ...
- ... auf den Kanister drücken
- langsam und vollständig einatmen
- Atem möglichst 10 Sekunden anhalten

Die nachfolgenden Beispiele dienen der exemplarischen, weitergehenden Illustration der vorliegenden Erfindung, ohne selbige auf deren Inhalt zu beschränken.

Formulierungsbeispiele (Zusammensetzung)

1 (Referenzbeispiel)

| | |
|---|---|
| - Tiotropiumbromid-Monohydrat | 0,04 Gew.% |
| - Zitronensäure | 0,05 Gew.% |
| - Wasser | 1,0 Gew.% |
| - Ethanol | 30,0 Gew.% |
| - HFA134a | 68,9 Gew.% |
| **Gesamt** | **100 Gew: % (13,5 g)** |

2.

| | |
|---|---|
| - Tiotropiumbromid-Monohydrat | 0,01 Gew.% |
| - Zitronensäure | 0,02 Gew.% |
| - Wasser | 1,5 Gew.% |
| - Ethanol | 15,0 Gew.% |
| - HFA134a | 83,5 Gew.% |
| **Gesamt** | **100 Gew: % (14,5 g)** |

3.

| | |
|---|---|
| - Tiotropiumbromid-Monohydrat | 0,02 Gew.% |
| - Zitronensäure | 0,04 Gew.% |
| - Wasser | 2,0 Gew.% |
| - Ethanol | 35,0 Gew.% |
| - BHT (Butylhydroxytoluol) | 0,1 Gew.% |
| - HFA134a | 62,8 Gew.% |
| **Gesamt** | **100 Gew: % (13,2 g)** |

4 (Referenzbeispiel)

| | |
|---|---|
| - Tiotropiumbromid-Monohydrat | 0,02 Gew.% |
| - Schwefelsäure | 0,1 Gew.% |
| - Wasser | 1,0 Gew.% |
| - Ethanol | 20,0 Gew.% |
| - HFA134a | 78,9 Gew.% |
| **Gesamt** | **100 Gew: % (14,2 g)** |

5 (Referenzbeispiel)

| | |
|---|---|
| - Tiotropiumbromid-Monohydrat | 0,04 Gew.% |
| - Salzsäure | 0,2 Gew.% |
| - Wasser | 0,5 Gew.% |
| - Ethanol | 15,0 Gew.% |
| - HFA134a | 84,3 Gew.% |
| **Gesamt** | **100 Gew: % (14,6 g)** |

## Patentansprüche

1. Inhalator enthaltend eine Arzneimittelformulierung zur Behandlung von entzündlichen oder obstruktiven Atemwegserkrankungen, wobei die Arzneimittelformulierung als Wirkstoff ausschließlich Tiotropiumbromid in gelöster Form enthält, **dadurch gekennzeichnet, dass** die Arzneimittelformulierung

    • das Treibgas HFA 134a
    sowie die weiteren Bestandteile
    • 0,005-0,03 Gew.-% des Arzneistoffs Tiotropiumbromid
    • eine Mischung aus den beiden Co-Solventien Ethanol und Wasser, wobei der Ethanol-Gehalt zwischen 15-40 Gew.-% und der Wassergehalt zwischen 0,5-2,5 Gew.-% beträgt, und
    • Zitronensäure in einer Konzentration zwischen 0,005-0,1 Gew.-%, enthält, wobei sich die Gew.-%-Angaben auf eine Dosierkammer von 50 µl beziehen, und **dadurch gekennzeichnet dass** die nominale Dosis der Einmalgabe zwischen 5,5 µg und 6,5 µg, die ausgebrachte Dosis zwischen 4,5-5,6 µg, und die Lungendosis (Fine Particle Dose) zwischen 2-3 µg und die mittlere aerodynamische Partikelgröße, bestimmt nach Pharm Eur 2.9.18 Apparatus D, zwischen 2,5-3,5 µm beträgt und wobei
    • die Dosis durch Abgabe einer Einzeldosis mittels Sprühstoß erfolgt, indem eine Dosis aus der Dosierkammer durch Betätigen des Inhalators durch Austritt aus der Düse ausströmt,
    • der Bohrungsdurchmesser des verwendeten Sprühkopfes einen Durchmesser zwischen 0,2-0,3 mm aufweist,
    • wobei die Dosierkammer ein Volumen zwischen 25 µl und 100 µl, bevorzugt 50 µl aufweist.

2. Inhalator enthaltend eine Arzneimittelformulierung zur Behandlung von entzündlichen oder obstruktiven Atemwegserkrankungen nach Anspruch 1, wobei die Dichtungsringe im Ventilkopf aus Ethylen-Propylen-Dien-Monomer (EPDM) hergestellt sind, und dass das gebildete Aerosol gekennzeichnet ist, dass die nominale Dosis der Einmalgabe zwischen 5,5µg und 6,5µg, die ausgebrachte Dosis zwischen 4,5-5,6 µg, und die Lungendosis (Fine Particle Dose) zwischen 2-3 µg und die mittlere aerodynamische Partikel größe zwischen 2,5-3,5 µm beträgt.

3. Inhalator enthaltend eine Tiotropiumbromid-haltige Arzneimittelformulierung nach einem der Ansprüche 1 bis 2 zur Verwendung bei der Behandlung von entzündlichen oder obstruktiven Atemwegserkrankungen.

## Claims

1. Inhaler containing a medicament formulation for the treatment of inflammatory or obstructive respiratory complaints, the medicament formulation containing as active substance exclusively tiotropium bromide in dissolved form, **characterised in that** the medicament formulation contains

    • the propellant gas HFA 134a as well as the additional ingredients
    • 0.005-0.03 % by weight of the active substance tiotropium bromide
    • a mixture of the two cosolvents ethanol and water, the ethanol content being between 15-40 % by weight and the water content being between 0.5-2.5 % by weight, and
    • citric acid in a concentration of between 0.005-0.1 % by weight, the percentages by weight relating to a metering chamber of 50 µl, and **characterised in that** the nominal dose of the single dosage is between 5.5 µg and 6.5 µg, the delivered dose is between 4.5-5.6 µg, and the pulmonary dose (Fine Particle Dose) is between 2-3 µg and the mean aerodynamic particle size, determined according to Pharm Eur 2.9.18 Apparatus D, is between 2.5-3.5 µm, and
    • the dosage being administered by the delivery of a single dose by a spray jet, in which a dose flows out of the metering chamber by actuation of the inhaler to expel it from the nozzle,

• the bore diameter of the spray head used having a diameter of between 0.2-0.3 mm,
• the metering chamber having a volume of between 25 µl and 100 µl, preferably 50 µl.

**2.** Inhaler containing a medicament formulation for the treatment of inflammatory or obstructive respiratory complaints according to claim 1, wherein the sealing rings in the valve head are made from ethylene-propylene-diene monomer (EPDM), and the aerosol formed is **characterised in that** the nominal dose of the single dosage is between 5.5 µg and 6.5 µg, the delivered dose is between 4.5-5.6 µg, and the pulmonary dose (Fine Particle Dose) is between 2-3 µg and the mean aerodynamic particle size is between 2.5-3.5 µm.

**3.** Inhaler containing a tiotropium bromide-containing medicament formulation according to either claim 1 or claim 2 for use in the treatment of inflammatory or obstructive respiratory complaints.

**Revendications**

**1.** Inhalateur contenant une formulation médicamenteuse pour le traitement de maladies respiratoires inflammatoires ou obstructives, dans lequel la formulation médicamenteuse contient comme principe actif exclusivement du bromure de tiotropium sous forme dissoute, **caractérisé en ce que** la formulation médicamenteuse contient

• le gaz propulseur HFA 134a
ainsi que les autres constituants
• 0,005-0,03 % en poids de la substance médicamenteuse bromure de tiotropium
• un mélange composé des deux co-solvants éthanol et eau, dans lequel la teneur en éthanol est comprise entre 15-40 % en poids et la teneur en eau entre 0,5-2,5 % en poids, et
• de l'acide citrique dans une concentration comprise entre 0,005-0,1 % en poids, dans lequel les indications de % en poids se rapportent à une chambre de dosage de 50 µl,
et **caractérisé en ce que** la dose nominale de la prise unique est comprise entre 5,5 µg et 6,5 µg, la dose distribuée entre 4,5-5,6 µg, et la dose pulmonaire (dose de particules fines) entre 2-3 µg et la taille de particules moyenne aérodynamique, déterminée selon Pharm Eur 2.9.18 appareil D, entre 2,5-3,5 µm et dans lequel
• la dose est obtenue par délivrance d'une dose individuelle au moyen d'une pulvérisation, du fait qu'une dose s'écoule de la chambre de dosage par sortie de la buse par actionnement de l'inhalateur,
• le diamètre de trou de la tête de pulvérisation utilisée présente un diamètre compris entre 0,2-0,3 mm,
• dans lequel la chambre de dosage présente un volume compris entre 25 µl et 100 µl, de préférence de 50 µl.

**2.** Inhalateur contenant une formulation médicamenteuse pour le traitement de maladies respiratoires inflammatoires ou obstructives selon la revendication 1, dans lequel les bagues d'étanchéité dans la tête de soupape sont fabriquées à partir d'éthylène-propylène-diène monomère (EPDM), et que l'aérosol formé est **caractérisé en ce que** la dose nominale de la prise unique est comprise entre 5,5 µg et 6,5 µg, la dose distribuée entre 4,5-5,6 µg, et la dose pulmonaire (dose de particules fines) entre 2-3 µg et la taille de particules moyenne aérodynamique entre 2,5-3,5 µm.

**3.** Inhalateur contenant une formulation médicamenteuse contenant du bromure de tiotropium selon l'une quelconque des revendications 1 à 2 destinée à être utilisée pour le traitement de maladies respiratoires inflammatoires ou obstructives.

Abbildung 1:

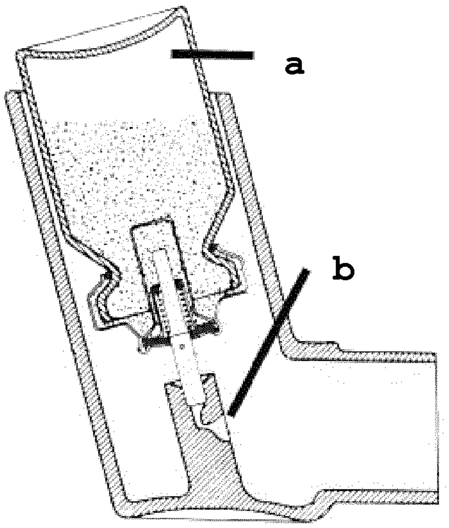


Abbildung 2:

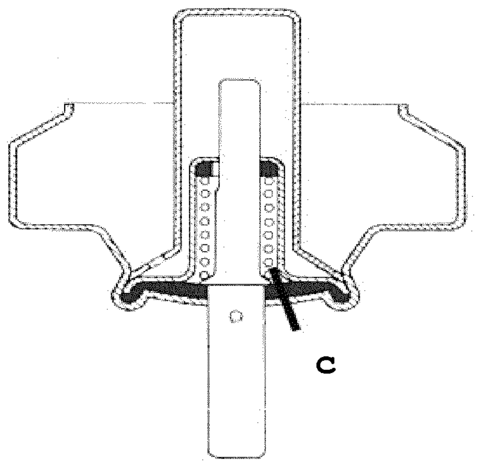

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0418716 A
- WO 0230389 A
- WO 03084519 A
- WO 03000241 A
- EP 2322243 A
- WO 2006002840 A
- WO 2005042528 A1


### In der Beschreibung aufgeführte Nicht-Patentliteratur


- Apparatus D, Andersen Cascade Impacter. Pharm. Eur. 2.9.18, European Pharmacopeia. 2008, vol. 2.9.18
- Interpretation of pH Measurments in Alcohol-Water Solvents. *J.Phys.Chem.,* September 1963, vol. 67, 1833-1838